# EUROPEAN PATENT SPECIFICATION

(11) **EP 1 491 099 B1**
(45) Date of publication and mention of the grant of the patent: **02.05.2012**
(21) Application number: 03710296.9
(22) Date of filing: 11.03.2003
(51) Int. Cl.: A23L 1/30

(54) **PROCESS FOR PRODUCING SDG**
VERFAHREN ZUR HERSTELLUNG VON SDG
PROCEDE DE PRODUCTION DE SDG

(30) Priority: 11.03.2002 JP 2002112687; 24.10.2002 JP 2002309152
(43) Date of publication of application: 29.12.2004
(73) Proprietor: Suntory Holdings Limited, Kita-ku, Osaka-shi Osaka 530-8203 (JP)
(72) Inventor: ABE, Keiichi, Ikeda-shi, Osaka 563-0023 (JP); IINO, Taeko, Otsu-shi, Shiga 520-0113 (JP); FUJII, Wataru, Ibaraki-shi, Osaka 567-0031 (JP); SUWA, Yoshihide, Ibaraki-shi, Osaka 567-0009 (JP)
(74) Representative: Vossius & Partner
(86) International application number: PCT/JP2003/002850
(87) International publication number: WO 2003/075686

(56) References cited:
- EP-A2- 0 906 761
- WO-A-02/062812
- WO-A-02/080702
- JP-A- 9 208 461
- US-B1- 6 498 145

## Description

### TECHNICAL FIELD

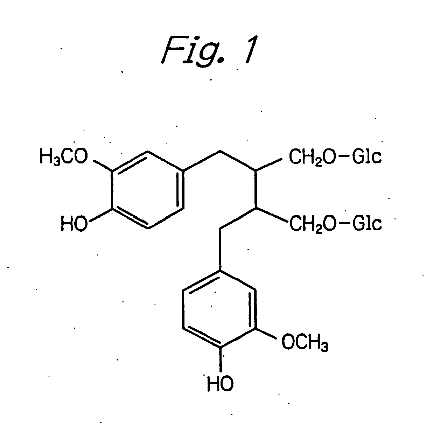

The present invention relates to a process for preparing SDG (secoisolariciresinol diglycoside, see FIG. 1), which is a lignan compound and known to be a precursor of compounds having female hormone-like activities such as enterolactone and enterodiol. Foods and drinks prepared by incorporating SDG are effective for various symptoms caused by imbalanced female hormones such as menopausal symptoms, osteoporosis, hyperlipidemia, obesity and depression.

### BACKGROUND ART

Climacteric syndrome is thought to include various symptoms caused by hormonal imbalance due to estrogen decline at the approach of the menopause and continue for several years until the menopause. Known symptoms characteristic of the climacteric include depression, hot flashes, etc. The climacteric precedes the menopause, which is associated with symptoms such as osteoporosis, hyperlipidemia, hypertension, obesity and depression.

These symptoms have serious effects on health, thus making desirable proper treatment. In addition to individual therapies selected for symptoms such as osteoporosis, hyperlipidemia, hypertension, obesity and depression, HRT or hormone therapy is also considered to be effective. Administration of female hormones often produces a dramatic improvement even in patients with more than one symptom.

However, HRT involves a risk of cancer such as breast and uterine; it also invites pseudomenstrual bleeding. It can be said that due to the deep-seated association of HRT with carcinogenicity and bleeding, in spite of the expected benefits and the fact that carcinogenicity seems to have been mostly eliminated by combined use with progesterone, there are still few cases wherein it is decided to use HRT.

It can be easy to imagine that foods with comparable effects to HRT would be highly advantageous. Under these circumstances, phytoestrogens have recently attracted attention in not only Japan but also the United States.

A typical plant estrogen is soybean isoflavone, which is a food material recently attracting attention. There are many findings that support the claim that soybean isoflavone is effective against various symptoms caused by imbalanced female hormones such as menopausal symptoms, osteoporosis, hyperlipidemia, obesity and depression because it is known to show a female hormone-like action. Soybean isoflavone is contained in soybean embryo extracts and collectively means genistein, daidzein and glycitin, most of which exist as glycosides and thought to be aglyconated by enteric microbes and absorbed.

Soybean isoflavone is expected to bind to estrogen receptors due to its structural properties common to estrogens, and it has been actually reported to selectively bind to estrogen receptor ERβ (Endocrinology 139, 4252(1998)).

The presence of phytoestrogens has been known for a long time, and they are known to make cows produce milk more efficiently; however, they also induce infertility if consumed in large quantities. Thus, their use requires due caution. Considering the safety for humans, empirically proved safety as a food component is most important.

It is known that the incidence of uterine cancer, breast cancer and male prostatic cancer is much lower and menopausal symptoms are milder in Japanese than Americans (J. Nutrition 125, 757S (1995)). A major reason for this is the isoflavone level in the blood, which is found to be about ten times or more higher in Japanese than Americans. Moreover, soybean isoflavone has been shown to have many effects. For example, it was shown to inhibit the growth of breast cancer and uterine cancer by experiments with cultured cells or animals and also shown to improve osteoporosis by controlling the bone resorption process or improve menopausal hyperlipidemia or to control obesity in menopausal model ovariectomized rats.

It can be concluded from these findings that soybean isoflavone is a plant estrogen expected to improve many symptoms including menopausal with high safety.

It is thought that the average intake of isoflavones in Japanese is about 20 mg daily. Positive ingestion of isoflavones is recommended and the Japan Health Food and Nutrition Food Association recommends a standard intake of 10~90 mg in so-called isoflavone-containing foods in relation to foods for isoflavone ingestion. In terms of foods for specified health use, isoflavones have the authorization of the Ministry of Health, Labor and Welfare as safe and effective materials on the basis of the bone resorption controlling effect.

In addition to isoflavones, many components called phytoestrogens are known, such as prenylnaringenin contained in hop, coumestrol contained in alfalfa, and recently highlighted lignan compounds contained in flax.

Geographic analysis shows that the major sources of phytoestrogens are isoflavones in the East in contrast to lignans in North Europe. Statistics show that both areas enjoy more benefits from consumption of plant estrogens than the other areas as evidenced by the lower incidence of breast cancer and the lower severity of menopausal symptoms.

It is considered that active components having a hormonal action require considerable caution when being ingested; researchers rank isoflavones and lignans as the two main phytoestrogens in the world as evidenced by the presence of findings showing the epidemiological utility of isoflavones and lignans.

A lignan compound contained in flax (flax lignan) was shown to be converted by enteric bacteria into active components showing a female hormone-like action such as enterolactone and enterodiol (Nature 298, 659 (1982)). It should be noted here that a study of Thompson et al. showed that lignans contained in flaxseeds such as SDG are converted into active components with high efficiency among lignan-containing plant materials (Flaxseed in Human Nutrition, Chapter 5 (pp. 82-98), academic press, AOCS Press (1995)).

In this manner, much attention is focused on flax lignan. Flax itself has been used for food since the Neolithic Age through the ancient Greek, Roman or Egyptian Age down to the present. However, there are difficulties in directly digesting flaxseeds due to their hard shells, high fat and high BTU, the inclusion of cyanogenic glycosides, and the presence of antagonists of vitamin B. In addition, flaxseed oil is susceptible to deterioration because of the high content of unsaturated fatty acids. Therefore, it has been used mostly for industrial oil but not attracted attention for use as a food in Japan

In Japan, it should be noted that the intake of lignan-type plant estrogens is low, even though the intake of isoflavones can be evaluated at a certain level from the intake of bean products in an ordinary dietary life. However, few foods rich in flax lignan are on the market, and it is desirable to develop food materials in which the abovementioned problems are solved.

On the other hand, a report proposes the use of lignan compounds contained in flax as food additives. JPA HEI-11-221048 describes that methods for relieving or preventing hot flashes, osteoporosis, sleep disorders, vaginal dryness and premenstrual syndrome using isoflavones, lignans, saponins, catechins and phenolic acids, and mentions that flax lignan as an example of lignans. Japanese Patent No. 306525 (JP 9208461) discloses lignan compounds as discoloration inhibitors, and mentions SDG as an example of lignan compounds. However, the physiological action of flax lignan is yet to be explained.

As for processes for preparing lignans, US Patent No. 5,705,618 relates to processes for extracting lignan from flaxseeds. This patent describes processes comprising alcohol extraction followed by concentration and alkali treatment. Japanese Patent No. 3065925 discloses processes in which alcohol extracts or raw lignan glycoside concentrates from flaxseeds are treated with an enzyme or an acid. However, these processes involve complex steps and still need improvements to reduce costs. Moreover, these processes provide only products with low amino acid contents, even though flaxseeds are known to be rich in protein.

### DISCLOSURE OF THE INVENTION

An object of the present invention is to develop an efficient process for preparing SDG (secoisolariciresinol diglycoside) known to be a precursor of compounds having female hormone-like activities such as enterolactone and enterodiol so that novel food materials containing SDG can be practically available.

Particularly, the present invention relates to a process for preparing a purified SDG-rich product comprising:
(a) extracting a plant material containing secoisolariciresinol diglycoside (SDG) with a basic alcohol; and
(b) purifying the obtained SDG-rich product on a styrenedivinylbenzene absorbent resin.

Furthermore, foods and drinks of any shape and category including healthy food and /or supplements, which contain the SDG-rich product obtained by the present invention and which are effective for preventing and relieving various symptoms caused by imbalanced female hormones (primarily, estrogen) such as menopausal symptoms, osteoporosis, hyperlipidemia, hypertension, obesity, depression and hot flashes are disclosed.

The inventors focused attention on phytoestrogens and carefully studied the physiological action of flax lignan, with the result that SDG contained in flax lignan was surprisingly found to be effective for various symptoms caused by imbalanced female hormones such as menopausal symptoms, osteoporosis, hypertension, hyperlipidemia, obesity, depression and hot flashes.

The inventors developed a process for efficiently preparing a lignan compound SDG contained in flaxseeds at high yield. In the present invention, a plant material containing SDG such as flaxseeds or defatted residues thereof is extracted with a basic alcohol. According to the present invention, it was surprisingly found that a lignan compound SDG can be recovered at high yield if the material is extracted with a basic alcohol and hydrolyzed simultaneously; material-derived amino acids can also be efficiently recovered.

Conventional processes in which extraction and hydrolysis separately take place substantially required three steps including a concentration step between the two steps. In contrast, the processes of the present invention allow the lignan compound to be extracted in one step, so that SDG can be prepared very efficiently.

### Brief Explanation of the Drawings

FIG. 1 shows the structural formula of SDG.
FIG. 2 shows changes in body weight in an antiobesity effect test.
FIG. 3 shows improvement in hot flashes and effect of combination with isoflavone.
FIG. 4 shows indefinite complaints-improving effect in menopausal females.

### THE MOST PREFERRED EMBODIMENTS OF THE INVENTION

The active component in the present invention SDG may be prepared from any natural sources containing the lignan, SDG. For example, lignan is contained at 2~8 mg/kg in whole-grain cereals such as oat, corn, rye, buckwheat and wheat. Flaxseeds are especially preferred as sources of SDG because they are reported to contain lignan at 800 mg/kg, which corresponds to 100 times that whole-grain cereals.

SDG may be prepared from flaxseeds, which are crushed and then defatted by extraction with a solvent suitable for food manufacturing such as hexane, more preferably defatted flax cake, i.e. residues from extraction of flaxseeds with hexane. Such defatted flax cake was usually discarded or used as diet for livestock, but not effectively used for food.

Two points should be noted about processes for preparing SDG from defatted flax cake. One is the presence of mucopolysaccharides contained in flaxseeds and the other is polymerization of lignan compounds via sugar chains. In view of these points, an efficient process for preparing SDG as developed in which decomposition and extraction take place at the same time.

First of all, suitable solvents include those normally used for extracting plant materials, especially those suitable as food materials. Alcohols for foods are most suitable, e.g. methanol, ethanol, propanol, isopropanol and butanol. Alcohols used for extraction must be prepared at a concentration that does not allow mucopolysaccharides to be extracted, preferably 30-100% (v/v), e.g. 50% (v/v) alcohol.

The extraction efficiency can be further improved by extracting SDG at the concentration shown above under alkaline conditions so as to hydrolyze sugar chains and extract unextracted SDG.

Thus, the basic alcohol extraction of the present invention, i.e. the processes combining hydrolysis under alkaline conditions with extraction, is industrially greatly advantageous because target components can be recovered more simply at higher yield. The processes of the present invention are also effective in that proteins contained in flaxseeds are extracted in the form of amino acids by basic alcohol extraction more efficiently than conventional processes.

The extraction solution is suitably used in amounts of 1-20 folds excess of flax cake. However, it may be used in smaller amounts in circulating extraction apparatuses rather than batch processes.

The alkali used in the basic alcohol extraction step may be of any type suitable for food manufacturing, e.g. NaOH.

The concentration of NaOH to be added is 0.05-2N NaOH, more preferably 0.1-0.2N, depending on the amount of flax cake to be treated.

Basic alcohol extraction can be completed in a shorter period than conventional alcohol extraction, within 30 minutes to 3 hours, typically about 1 hour at room temperature. The extraction temperature is preferably the boiling point or less of alcohol, e.g. room temperature to 70°C when a conventional extraction apparatus is used. The treatment can be performed at higher temperatures if a pressurizable extraction apparatus is used.

Extracts must be finally neutralized with an acid, preferably an acid suitable for food manufacturing such as acetic acid and hydrochloric acid. The acid is added in an amount necessary to neutralize the extracts.

1-2 weigh% SDG to raw materials weight can be extracted with a basic alcohol of the present invention. The SDG-rich product contains amino acids such as glutamic acid expected to be effective for recovery from fatigue and improvement of vitality; and valine, isoleucine and leucine presumably taken up into muscles to directly provide a source of energy in amounts about 8~10 times greater than SDG-rich products extracted by conventional techniques. Therefore, the SDG-containing composition can be ingested as it is or be in the form of a food or drink or as an additive in a food or drink to provide benefits for recovery from fatigue and improvement of vitality in addition to those from SDG. This SDG-rich product can be directly used as a food material after desalting because it is substantially free from cyanogenic glycosides. It can be further purified into a richer food material in a purification process to increase the purity. Any purification techniques suitable for food manufacturing can be applied, such as the use of activated carbon, silica gel, reverse-phase resins (ODS) and styrenedivinylbenzene adsorbent resins (Diaion HP-20, Mitsubishi Chemical Industries, Ltd.), or non-resin techniques such as CPC (centrifugal liquid-liquid separation), crystallization or differentiation based on the difference of the solubility in a solvent.

To suit the intended purpose, the SDG content can be increased to 10%-90% or more per solid, depending on the conditions used. Especially high-purity samples can be prepared by repeating preparative HPLC column chromatography on ODS.

According to the process of the present invention, SDG can be incorporated into foods/drinks as a lignan compound effective for health maintenance by degrading viscous materials contained in flaxseeds that were difficult to incorporate into foods/drinks due to the presence of such viscous materials.

Thus obtained SDG-rich product can preferably be dried into powder by standard techniques such as vacuum concentration, spray drying or freeze-drying.

Novel SDG-rich food materials extracted by the processes of the present invention are disclosed. The SDG-rich food materials obtained by the present invention are colorless, odorless and tasteless and highly soluble in water at or below 40 weight % SDG, so that they can be eaten alone as powdery foods or in powdery foods such as soy flour or can be used as food additives to prepare various foods and drinks.

Oral compositions that allow a necessary intake of SDG by incorporating SDG are disclosed.

The SDG-containing foods/drinks were found to be especially suitable for preventing or relieving various symptoms caused by imbalanced female hormones, especially estrogens. The SDG-containing foods can be prepared so that they contain SDG in an amount corresponding to a daily intake of 1 mg-1000 mg, more preferably 5 mg-500 mg, most preferably 10-250 mg. The dose of SDG is selected to suit the intended purpose of the food, for example 10 mg-90 mg for use in foods having a form suitable for continuous ingestion on a daily basis. However, foods can contain doses of up to 500 mg, for example, when more healthy effects are desired.

The SDG containing ratio is selected to be relatively higher in food forms with low water content such as powdery foods or tablets or in products themselves consumed in small amounts. Foods containing e.g. 0.1%-90% by weight of SDG can be developed, taking into account the intake varying with the purpose.

In the case of products having a high water content and themselves consumed in large amounts such as drinks and retort foods, the SDG content can be appropriately decreased, preferably in the range of 0.001%-10% by weight, for example.

SDG-rich products can be used as materials for regular foods as well as functional foods and dietary supplements. Examples of such foods are solid foods such as bread, biscuits and cereal bars; and liquid foods such as juice and soup. Functional foods may be in a processed form such as powder, granule, tablet, soft capsule, hard capsule, internal medicine and syrup. They can be prepared by conventional processes.

When tablets are prepared, they can contain any conventional excipients including, but not limited to, cellulose, lactose, dextrin, powder sugar, corn starch, reduced maltose and sucrose fatty acid ester. They may be coated with any material suitable for coating including, but not limited to, shellac, carnauba wax and sugar for moisture resistance or other purposes. Other functional components may be added, e.g. antioxidant materials such as vitamin E and polyphenol and minerals such as calcium.

They can also be used in drinks for healthy purposes because of their high solubility in water, their taste and odor having no significant influence on formulations. They can be flavored into various forms such as tea, soft drinks and health drinks (including powdered health drinks). In the case of regular drink forms, a recommended dose of 0.01 mg/ml-10 mg/ml can be selected. In the case of concentrated drinks, the maximum dose can be increased depending on the concentration degree.

No sufficient data have so far existed about the comparison of effectiveness between SDG and isoflavone. Comparison tests of their activities were performed to show that SDG has an efficacy comparable or superior to isoflavone and that combination of both produces more significant effects. Foods and drinks in which SDG is combined with isoflavone, are very effective for various symptoms caused by imbalanced female hormones such as climacteric syndrome, osteoporosis, hyperlipidemia, hypertension, obesity, depression and hot flashes can be provided.

Isoflavone is added to provide a daily intake of 10∼90 mg, for example.

### EXAMPLES

The following examples further illustrate the present invention.

### Example 1

### Extraction method

To a suspension of 1 kg of commercially available flax cake in 10 L of 50% ethyl alcohol was added 400 ml of 4N NaOH. The temperature was raised to 50°C with stirring, and the mixture was extracted for 1 hour. The extract was neutralized with acetic acid and then filtered and concentrated under reduced pressure to give 242 g of a basic alcohol extract (composition 1).

For comparison, an extract was prepared without adding NaOH, i.e. 100 g of flax cake was extracted with 1 L of 50% ethyl alcohol at 50°C for 4 hours, and then the extract was filtered and combined with 40 ml of 4N NaOH and then degraded for 4 hours at 50°C. After neutralization, concentration under reduced pressure gave 11.1 g of an extract (composition 2).

### Example 2

### SDG analysis method and results

The amount of SDG extracted in the above compositions 1 and 2 were compared by HPLC analysis.

The analysis was performed on an ODS column (Deverosil 5HG-3, 4.6 x 50 mm, Nomura Chemical Co., Ltd.) in the presence of 0.1% TFA (trifluoroacetic acid) at a flow rate of 1 ml/min under gradient conditions of 10% → 80% acetonitrile over 8 minutes. The column was monitored with a UV detector at a wavelength of 280 nm and quantitatively analyzed on the basis of peak heights.

Samples were tested in water solution.

The results are shown in Table 1.

Under these analytical conditions, SDG can be detected as a peak appearing at a retention time of 3.8 minutes.

It was shown that the yield was about 20% higher in composition 1 extracted with a basic alcohol than composition 2 extracted with an alcohol.

**Table 1 Increase in recovery by basic alcohol extraction**

| | | Material | Solids recovered | SDG content | |
|---|---|---|---|---|---|
| | Basic alcohol extraction | 1 Kg | 242 g | 15 g | |
| | | | | | |
| | Alcohol extraction | 100 g | 11.1 g | 1.3 g | |
| | | | | | |

### Example 3

### Amino acid analysis and results

Flaxseeds are known to be rich in protein, but it seems difficult to extract at high concentrations of alcohol. Thus, an amino acid analysis was performed to examine whether or not the amino acid content can be increased by basic alcohol treatment, using a high-speed amino acid analyzer Hitachi amino acid analysis system L8800 (OPA method).

The results are shown in Table 2. The novel process based on basic alcohol extraction achieved higher contents of amino acids that were extracted in only small amounts by conventional techniques, especially an 8.1-fold increase in the extraction of glutamic acid which can be expected to be effective for recovery from fatigue and improvement of vitality and about 8 to 11-fold increases in the contents of amino acids presumably taken up into muscles to directly provide a source of energy such as valine, isoleucine and leucine, showing that these active ingredients can be efficiently recovered by the process of the present invention and thus demonstrating the utility of the process of the present invention.

**Table 2 increase in amino acid recovery by basic alcohol extraction**

| | | Amounts of amino acids extracted per g of flax cake (mg) | | | | |
|---|---|---|---|---|---|---|
| | | Glutamic acid | valine | Isoleucine | Leucine | |
| | Basic alcohol extraction | 22 | 38 | 33 | 47 | |
| | | | | | | |
| | Alcohol extraction | 2.7 | 3.6 | 4.2 | 4.3 | |
| | Comparison (ratio) | 8.1 | 10.6 | 7.9 | 10.9 | |

### Example 4

### HP20 purification

242 g solution of composition 1 obtained in Example 1 (containing 15g of SDG) dissolved in 2 L of water was applied on Diaion HP-20 (8 cmφ x 60 cm column). After washing in 8 L of water, the column was eluted with 5 L of 40% ethanol (v/v) at a flow rate of 1 vvm to give a fraction of 39 g (composition 3) containing 14.3 g of SDG.

Example 1 and the operation described above for composition 3 were repeated to give fractions of 95 g (containing 36.6 g SDG), which were dissolved in 500 ml of water and applied on Diaion HP-20 (8 cmφ x 40 cm column). After washing withwater, the column was stepwise eluted with 5 L each of 10%, 15%, 20% and 40% ethanol (v/v) at a flow rate of 1 vvm.

The fractionation results are shown in Table 3. SDG was scarcely eluted with 10% ethanol. Subsequent elution with 15% and 20% ethanol recovered 14.1 g and 16.6 g of SDG at purities of 74.2% and 91.0%, respectively. Then, elution with 40% ethanol recovered 2.9 g of SDG at a purity of 11.8%. No more SDG was eluted even when the ethanol concentration was further increased.

These results show that high-purity SDG can be prepared using HP20. For example, SDG is preferably eluted at an alcohol concentration of 15% or more.

The fraction eluted with 20% ethanol (composition 4) in this example was subjected to efficacy tests.

**Table 3 Fractionation on HP20**

| | Concentration of eluting alcohol | Solids (g) | SDG (g) | SDG content (%) | |
|---|---|---|---|---|---|
| | 10% ethanol | 18 | 1.1 | 6.1 | |
| | 15% ethanol | 19 | 14.1 | 74.2 | |
| | 20% ethanol | 18.25 | 16.6 | 91.0 | |
| | 40% ethanol | 24.5 | 2.9 | 11.8 | |
| | Total | 79.75 | 34.7 | 43.5 | |
| | | | | | |

### Example 5

### Efficacy 1: Effects on lipids and bones and effects of combination with isoflavone

### Studies on estrogen-like action using menopausal model animals

It is known that after the menopause bone mass decreases through the activation of osteoclasts due to estrogen decline and causes osteoporotic symptoms. Estrogen decline may disturb the balance of lipid metabolism to cause symptoms such as hyperlipidemia and hypercholesterolemia. Ovariectomized female rats are commonly used as model animals of menopausal symptoms because ovariectomy induces the above symptoms due to the decline of estrogen levels. Experiments were performed using ovariectomized rats to demonstrate the effect of SDG controlling these menopausal symptoms by compensating for estrogen loss.

### Preparation of osteoporosis model rats by ovariectomy:

Wistar-strain female rats (Charles River Japan, Inc.) were used, among which 34 animals were ovariectomized (OVX) at 6 weeks of age and 9 animals were sham-operated (Sham) at the same age, and raised on not-purified chow (CE 2; Clea Japan). After 2 weeks, the ovariectomized rats were divided into 4 groups (n = 8∼9) called groups I to IV. The sham-operated non-ovariectomized group (group V, n = 9) was used as a positive control.

### Raising of ovariectomized rats:

Group I and group V were free access for 4 weeks to a basic diet consisting of AIN-93M Mix purified diet without Ca (Oriental Yeast Co., Ltd.). Group II, group III and group IV were fed with the basic diet supplemented with 0.1% each of soybean isoflavone (containing 80%), composition 4 (containing 91% SDG), and a combination of soybean isoflavone and composition 4, respectively. On the end date of raising the rats, they were weighed and then anatomized to collect blood and dissect the right femur.

### Evaluation of inhibitory effect on bone mass decline:

After adherent tissues were removed, the femur dissected from the rat's right leg was dried at 100°C for 24 hours and weighed. As a result, the femoral weight per 100 g of rat body weight in group I markedly decreased as compared with group V, while group II, group III and group IV showed the tendency of inhibiting bone mass decline as compared with group I (Table 4).

**Table 4 Bone mass in ovariectomized rats**

| | Femoral dry weight (mg) (per 100 g body weight) |
|---|---|
| Group I | 87.9±1.3 |
| Group II | 89.5±1.8 |
| Group III | 90.9±2.7 |
| Group IV | 93.1±2.6 |
| Group V | 112.8±1.4 |

Evaluation of lipid metabolism-improving effect: Plasma was isolated from rat blood and assayed for triglyceride by an automatic analyzer (Hitachi). In contrast to a marked increase in triglyceride levels in group I, group II showed an inhibitory tendency and group III and group IV showed significant inhibitory effect (Table 5).

**Table 5 Blood triglyceride levels in ovariectomized rats**

| | Blood triglyceride (mg/dl) |
|---|---|
| Group I | 50.3±7.5 |
| Group II | 38.6±3.5 |
| Group III | 32.0±3.2 * |
| Group IV | 28.9±3.1 ** |
| Group V | 24.7±1.9 ** |

| | |
|---|---|
| (*:p<0.05, **:p0.01 vs. group I) | |

The above test examples show that SDG has an effect on lipids and bones and that this effect increases by combination with isoflavone, i.e. combination of both is effective.

### Example 6

### Efficacy 2: Antiobesity effect

Female Wister-strain rats at 7 weeks of age were ovariectomized and treated with each sample once a day by forced oral administration using a metal gastric tube for 14 days after surgery. Each group consisted of 8 animals (n = 8).

The animals were weighed to evaluate the effect on obesity due to increased appetite after the menopause. The samples used were 17β estradiol as a positive control and genistein as a reference sample, which is a major compound of soybean isoflavone. The SDG sample was composition 4.

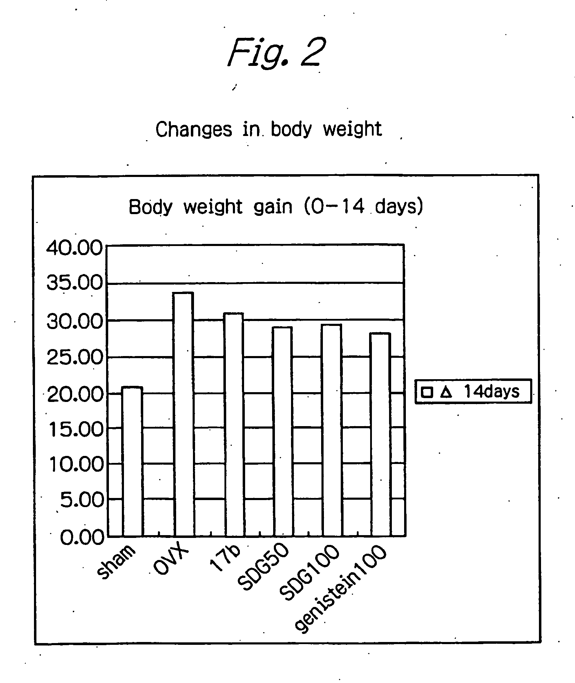

The results are shown in FIG. 2. Changes in body weight show that body weight gain was inhibited by intake of 50 mg/Kg SDG, 17β estradiol (0.02 mg/kg) and 100 mg/Kg genistein.

### Example 7

### Efficacy 3: Improvement in hot flashes and synergism with isoflavone

### Tests on menopausal model animals

A sharp decline of estrogen levels after the menopause invites hormone imbalance and autonomic failure to cause vasomotor disturbance-like symptoms such as hot flashes. In experimental animals, ovariectomy of female rats induces a rise in the temperature at the tail skin and symptoms corresponding to hot flashes among menopausal symptoms. The temperature at the tail skin was measured in ovariectomized rats treated with SDG and soybean isoflavone.

### Preparation of osteoporosis model rats by ovariectomy:

Wistar-strain female rats (Charles River Japan, Inc.) were used, among which 41 animals were ovariectomized at 6 weeks of age and 9 animals were sham-operated at the same age, and raised on not-purified chow (CE 2; Clea Japan). After a week, the ovariectomized rats were divided into 5 groups (n = 7∼9) called group I to V. The sham-operated non-ovariectomized group (group V, n = 9) was used as a positive control.

### Raising of ovariectomized rats:

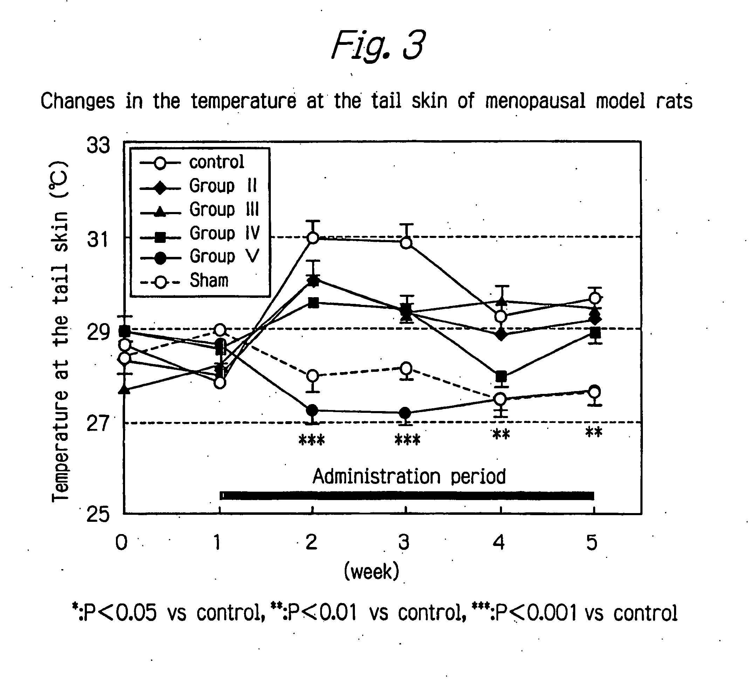

Group I and group V and non-ovariectomized group had free access for 4 weeks to a basic diet consisting of AIN-93M Mix purified diet without Ca (Oriental Yeast Co., Ltd.). Group II, group III and group IV were fed with the basic diet supplemented with 0.1% each of soybean isoflavone, SDG (composition 4), and a combination of soybean isoflavone and SDG, respectively. Group V was treated with 17β estradiol at 1 µg/day by subcutaneous administration through the period of ingesting the test diet. During the raising period, the temperature at the tail skin of the rat was measured with a precision thermometer BAT-12R (Physitemp). Ovariectomy induces a rise in the temperature at the tail skin of the rat, but the rise was inhibited by ingestion of isoflavone and SDG. This effect became more remarkable by combination of both, showing that combination of both is effective (FIG. 3).

### Efficacy 4: Influence of SDG and isoflavone on uterine weight

At the end of the studies in Example 7, the rats were autopsied and measured for uterine weight. Ovariectomy induced uterine atrophy and uterine weight loss, but group V treated with 17β estradiol did not show uterine atrophy with the weight being comparable to that of non-ovariectomized group. However, groups II to IV were comparable to group I and showed no effect of inhibiting uterine atrophy.

**Table 6 Uterine weight in ovariectomized rats**

| | Uterine weight (mg) |
|---|---|
| Group I | 93.7 ± 3.9 |
| Group II | 95.1 ± 6.4 |
| Group III | 105.5 ± 6.8 |
| Group IV | 100.7 ± 4.3 |
| Group V | 443.9 ± 28.8 *** |
| Non-ovariectomized group | 524.3 ± 71.4 *** |

| | |
|---|---|
| ***P<0.001 vs. group I | |

### Example 8

### Efficacy 5: Indefinite complaints-improving effect in menopausal females

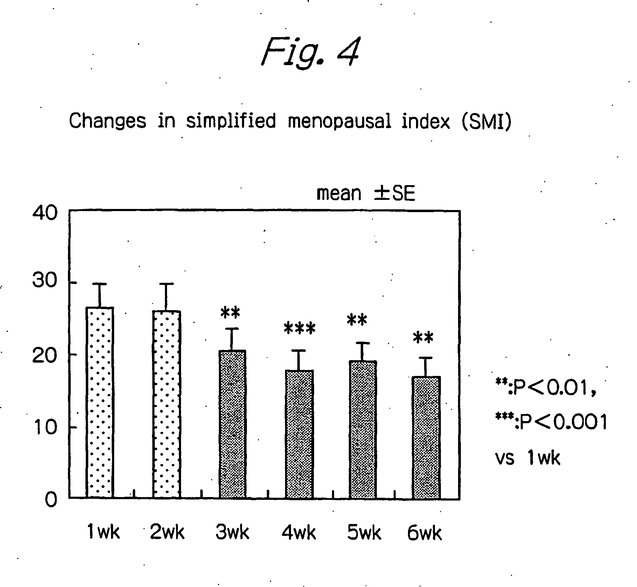

Test tablets containing 3.3 weight% of composition 4, 6.7 weight% of soybean isoflavone and 0.5 weight% of vitamin E were prepared by a standard method. Thirty-three females suffering from climacteric-specific indefinite complaints associated with the menopause were asked to ingest 3 test tablets (about 600 mg) daily for 4 weeks and to answer a questionnaire about changes in various symptoms according to the Kupperman menopausal index. The results of the questionnaire showed a significant improvement in vertigo, headache, melancholy, fatigue, arthralgia and muscle ache, as well as improving tendency in vasomotor disorder-like symptoms such as hot flashes and cold flashes. The simplified menopausal index (SMI) in each monitor significantly decreased as compared with the period before ingestion (FIG. 4).

### Example 9

### Preparation of SDG-containing tablets

Each powder having the composition shown in Table 7 was homogeneously mixed in a mixer and compressed into pellets each weighing 200 mg in a tabletting machine. Then, the pellets were sprayed with shellac on a coating pan and dried to prepare tablets.

**Table 7**

| | Formula 1 | Formula 2 | Formula 3 |
|---|---|---|---|
| Ingredient | Composition (%) | Composition (%) | Composition (%) |
| Extract 3 | 30 | 8 | 15 |
| Soybean isoflavone (30%) | | 22 | 15 |
| Vitamin E | 9 | | 5 |
| Lactose | 18 | 25 | 25 |
| Cellulose | 5 | 34 | 29 |
| Reduced maltose | 15 | | |
| Calcium | 14 | 8 | 8 |
| Corn starch | 5 | | |
| Sucrose fatty acid ester | 4 | 3 | 3 |
| Total | 100 | 100 | 100 |

### Example 10

### Preparation of SDG-containing bread

In a bowl, 1 g of the extract containing 40% SDG of the present invention was mixed with 3 g of dry yeast, 500 g of flour, 50 g of sugar, an egg, 2 g of salt and 300 ml of water and the mixture was thoroughly kneaded. The dough was fermented for 15 minutes, followed by degassing and fermentation for further 15 minutes. After degassing, the dough was divided into 20 equal round pieces, which were subjected to secondary fermentation for 1 hour on a baking sheet greased with salad oil and then baked in an oven heated at 180°C for 20 minutes to prepare bread.

### Example 11

### Drink formulation

A solution 1 g of sodium citrate, 200 g of glucose and 1 g of the extract containing 80% SDG dissolved in 20 L of drinking water was adjusted to pH 4.0 with citric acid and then dosed with citrus flavor and packed in a 120 ml brown bottle to prepare a drink formulation.

### INDUSTRIAL APPLICABILITY

According to the process of the present invention, novel food materials rich in SDG (secoisolariciresinol diglycoside) known to be a precursor of compounds having female hormone-like activities such as enterolactone and enterodiol as well as foods and drinks in various forms containing them can be prepared.

The development of such SDG-containing foods and drinks, especially foods and drinks containing SDG in combination with isoflavone can be expected to confer a great benefit on health because they can be expected to improve various symptoms caused by imbalanced female hormones such as menopausal symptoms, osteoporosis, hyperlipidemia, hypertension, obesity, depression and hot flashes or to provide cosmetic effects.

## Claims

1. A process for preparing a purified SDG-rich product comprising:
(a) extracting a plant material containing secoisolariciresinol diglycoside (SDG) with a basic alcohol; and
(b) purifying the obtained SDG-rich product on a styrenedivinylbenzene absorbent resin.

2. The process for preparing a purified SDG-rich product of claim 1 wherein the plant material containing SDG is flaxseed.

3. The process for preparing a purified SDG-rich product of claim 2 wherein the flaxseed is defatted flax cake

4. The process for preparing a purified SDG-rich product of any one of claims 1 to 3 wherein the alcohol has a concentration of 30-100% (v/v).

5. The process for preparing a purified SDG-rich product of any one of claims 1 to 4 wherein the basic alcohol contains 0.05-2N alkali.

6. The process for preparing a purified SDG-rich product of any one of claims 1 to 5 wherein the basic alcohol has a concentration of 30 to 50 % (v/v).

7. The process for preparing a purified SDG-rich product of any one of claims 1 to 6 wherein the alcohol is ethanol.

8. The process for preparing a purified SDG-rich product of any one of claims 1 to 7 wherein the extraction temperature is the boiling point of the alcohol or lower.

## Patentansprüche

1. Ein Verfahren zur Herstellung eines gereinigten SDG-reichen Produkts, umfassend:
(a) Extrahieren eines Pflanzenmaterials, das Secoisolariciresinol-Diglycosid (SDG) enthält, mit einem basischen Alkohol; und
(b) Reinigen des erhaltenen SDG-reichen Produkts auf einem Styroldivinylbenzol-Absorptionsharz.

2. Das Verfahren zur Herstellung eines gereinigten SDG-reichen Produkts gemäß Anspruch 1, wobei das SDG-enthaltende Pflanzenmaterial Leinsamen ist.

3. Das Verfahren zur Herstellung eines gereinigten SDG-reichen Produkts gemäß Anspruch 2, wobei der Leinsamen ein entfetteter Lein-Kuchen ist.

4. Das Verfahren zur Herstellung eines gereinigten SDG-reichen Produkts gemäß einem der Ansprüche 1 bis 3, wobei der Alkohol eine Konzentration von 30-100% (V/V) aufweist.

5. Das Verfahren zur Herstellung eines gereinigten SDG-reichen Produkts gemäß einem der Ansprüche 1 bis 4, wobei der basische Alkohol 0,05 - 2N Alkali enthält.

6. Das Verfahren zur Herstellung eines gereinigten SDG-reichen Produkts gemäß einem der Ansprüche 1 bis 5, wobei der basische Alkohol eine Konzentration von 30 bis 50% (V/V) aufweist.

7. Das Verfahren zur Herstellung eines gereinigten SDG-reichen Produkts gemäß einem der Ansprüche 1 bis 6, wobei der Alkohol Ethanol ist.

8. Das Verfahren zur Herstellung eines gereinigten SDG-reichen Produkts gemäß einem der Ansprüche 1 bis 7, wobei die Extraktionstemperatur dem Siedepunkt des Alkohols entspricht oder niedriger ist.

## Revendications

1. Procédé pour préparer un produit riche en SDG purifié comprenant :
(a) l'extraction d'un matériau végétal contenant du sécoisolaricirésinol diglycoside (SDG) avec un alcool basique ; et
(b) la purification du produit riche en SDG obtenu sur une résine absorbante en styrène-divinylbenzène.

2. Procédé pour préparer un produit riche en SDG purifié selon la revendication 1, dans lequel le matériau végétal contenant du SDG est la graine de lin.

3. Procédé pour préparer un produit riche en SDG purifié selon la revendication 2, dans lequel la graine de lin est un gâteau de lin dégraissé.

4. Procédé pour préparer un produit riche en SDG purifié selon l'une quelconque des revendications 1 à 3, dans lequel l'alcool a une concentration de 30 à 100 % (v/v).

5. Procédé pour préparer un produit riche en SDG purifié selon l'une quelconque des revendications 1 à 4, dans lequel l'alcool basique contient un alcali 0,05-2 N.

6. Procédé pour préparer un produit riche en SDG purifié selon l'une quelconque des revendications 1 à 5, dans lequel l'alcool basique a une concentration de 30 à 50 % (v/v).

7. Procédé pour préparer un produit riche en SDG purifié selon l'une quelconque des revendications 1 à 6, dans lequel l'alcool est l'éthanol.

8. Procédé pour préparer un produit riche en SDG purifié selon l'une quelconque des revendications 1 à 7, dans lequel la température d'extraction est égale ou inférieure au point d'ébullition de l'alcool.
